# EUROPEAN PATENT APPLICATION

(11) **EP 4 403 622 A1**
(43) Date of publication of application: **24.07.2024**
(21) Application number: 23152054.5
(22) Date of filing: 17.01.2023
(51) Int. Cl.: C12M 3/00, C12M 1/00, C12M 1/36

(54) **SMART CONTAINER FOR PERFORMING A BIOPROCESS AND CONTAINING BIOLOGICAL MATERIAL, IN PARTICULAR LIQUID IMMUNE CELL CULTURES**

(71) Applicant: The Automation Partnership (Cambridge) Ltd., Hertfordshire SG8 5WY (GB)
(72) Inventor: Stacey, Adrian, Cambridge CB4 1FY (GB); Prouté, Fanny, 37083 Göttingen (DE); Betts, John, London N4 4BX (GB); Pilgrim, James, Cambridge CB5 8PJ (GB)
(74) Representative: Gottschald Patentanwälte Partnerschaft mbB

(57) **Abstract**

The invention relates to a smart container for performing a bioprocess and containing biological material, in particular liquid immune cell cultures, wherein the smart container (2) comprises a base (3), wherein the smart container (2) comprises on the base (3) a storage structure (5), in particular fluidic storage structure (5), for the biological material, wherein the smart container (2) comprises on the base (3) a control unit (7) with a processor (8) and a memory (9), wherein a workflow being part of the bioprocess is stored in the memory (9), wherein an identifier of the biological material is stored in the memory (9), wherein the workflow comprises a step with a user action performed by a user. It is proposed that the smart container (2) comprises a local output arrangement (11) controlled by the control unit (7), that the processor (8) of the control unit (7) generates a user output to guide the user in performing the user action and that the control unit (7) outputs the user output via the local output arrangement (11).

## Description

The present invention relates to a smart container for performing a bioprocess and containing biological material, in particular liquid immune cell cultures according to the general part of claim 1, to an arrangement of at least two smart containers according to the general part of claim 14 and to an integrated bioprocessing system for performing a bioprocess on liquid immune or naive cell cultures according to claim 15.

The term "bioprocess" presently represents a biotechnological process, in particular a biotechnological process involving the use of immune cell cultures or naive cell cultures, in particular stem cell cultures. One or more processing steps might be performed on each cell culture. Hence, a bioprocess in this sense might refer to a manufacturing process that involves a sequence of processing steps performed on a cell culture which ultimately will lead to a final product.

Exemplarily, the bioprocess may be in the area of cell and gene therapy, for example to manufacture autologous T cells that are modified to express a chimeric antigen receptor (CAR). These cells might be used for the treatment of various types of hematologic malignancies, including different types of leukemia (blood cancer). Other cell therapies based on naive cells, in particular stem cells and their derivates are also of interest.

For biotechnological processes involving the use of liquid immune or naive cell cultures the starting material usually is quite heterogeneous regarding its composition, for example each donor or patient may be in a different condition (e.g., in terms of disease progression, genetic make-up or the immune system history). Therefore, certain steps of the bioprocess need to be flexibly adapted and tailored to each individual cell culture. Furthermore, a bioprocess involving the genetic modification of an immune cell culture will require a sequence of processing steps that is different from the sequence of processing steps required in a bioprocess not involving the genetic modification of an immune cell culture.

Hence, depending on the features of the cell culture and the bioprocess to be performed, various parameters, including for example the type, sequence or configuration of processing steps performed on each immune cell culture will need to be adjusted to the individual features of the cell culture. Further, the cell cultures are typically processed by different devices or need to be transferred between different locations to be processed. This may require an operator to pre-configure or adjust different devices to each cell culture, including the preconfiguration or reconfiguration of single-use components like tubes or tube-sets. Accordingly, different tubes may be connected differently or need to be installed into certain device components manually when performing the bioprocess.

In many bioprocesses, including the area of cell and gene therapy which may comprise allogenic or autologous production of genetically modified immune cells, compliance of the process with regulatory demands is important. To ensure the safety of the product, the manufacturing process is typically highly regulated by regulatory authorities.

Particular challenges arise, when a user needs to perform steps of the bioprocess and when the access to information may be time-critical and decisions need to be made within a certain time window to ensure compliance with regulatory demands or, especially for digital approaches, when the communication to a central management or database is lost.

In a highly regulated process, as stated above, any operator involvement required poses a threat to the product, because manual steps to be performed may be error-prone, for example if tube connections are established wrongly, or the sterility of the product may become endangered, if the wrong components are handled. However, complex processes can only be automated to a certain degree and critical decisions should ultimately be made by knowledgeable operators. Accordingly, to mitigate these risks, operators need to be trained extensively, a variety of measurements need to be taken, and an extensive documentation is mandatory to ensure the safety of the product. In this regard, user support is not just a question of working efficiently, but can also be a decisive step towards product quality and safety.

Bioprocesses, in particular production processes in the field of cell and gene therapy, usually involve a plurality of process steps. Accordingly, the cell culture is usually transferred, manually or automatically, between different devices. Further, the cell culture may also be transferred from and to different liquid containers and usually numerous reagents and equipment are utilized. The resulting data, including the type and quantity of materials used, process conditions and any deviations must be documented, and the documentation must be assigned to the final product in a suitable manner.

Recently, more and more integrated bioprocessing systems are proposed. Such integrated bioprocessing systems allow simultaneous performance of several bioprocesses on different cell cultures, wherein the bioprocesses or rather the processing steps to be performed can be adjusted according to the respective cell culture. However, ensuring that data are assigned to the correct bioprocess is a challenge within these systems, especially, when multiple bioprocesses are carried out in parallel. Further, even though operator involvement may be considerably reduced in these systems, whenever an operator needs to be involved, it needs to be ensured as much as possible that the required actions are performed without errors and correctly for each individual bioprocess.

While the named challenges are particularly relevant in bioprocesses carried out under GMP conditions, they may also arise in other bioprocesses where user interaction is necessary.

The known prior art which builds the basis of the invention (US 2022/283197 A1) concerns a smart container for performing a bioprocess and containing biological material, wherein the smart container comprises a base, wherein the smart container comprises on the base a storage structure for the biological material, wherein the smart container comprises on the base a control unit with a processor and a memory, wherein a workflow being part of the bioprocess is stored in the memory, wherein an identifier of the biological material is stored in the memory, wherein the workflow comprises a step with a user action performed by a user. The user action may be transporting the smart container to a device for performing a part of the bioprocess.

Although the decentralized approach already brings decisive advantages, it remains a problem that a user needs to perform, possibly time-critical, steps. It remains a challenge to decrease potential errors by human interaction.

The invention is based on the problem of improving the known smart container such that a further optimization regarding the named challenge is reached.

The above-noted object is solved by the features of the characterizing part of claim 1.

The main realization of the present invention is that user support and data access can be enhanced, when the data are output locally at the smart container. By including the user in this local routine and outputting a user output guiding the user during the performance of a user action locally, the advantages of doing some data processing, data integrity checking, workflow control, documentation and the like, locally, are enhanced. Even with a central workflow management system involved, outputting the user output locally allows easier access to information by the user and decreases potential errors.

The term "base" relates to a common carrier structure of the smart container. The base may be a single piece or composed of multiple, possibly separable, pieces. At least when assembled to be the smart container, the base allows handling of the smart container as such. The elements arranged on the base, which may also mean that the elements are part of the base, can be handled together with the base and are preferably attached to the base. The attachment may be permanent or temporary.

In detail, it is proposed that the smart container comprises a local output arrangement controlled by the control unit, that the processor of the control unit generates a user output to guide the user in performing the user action and that the control unit outputs the user output via the local output arrangement.

A user output is an output that can be understood by a user. The user output is output locally.

In embodiments according to claim 2, the local output arrangement is arranged on the base or temporarily connected to the base. Preferred embodiments relate to a screen and/or LEDs which may be arranged on the base or near a target of the user action. A screen has the advantage of being able to display complex information right at the smart container. LEDs are easy to place anywhere. For a good user guidance, for example, the screen may display "disconnect the tube from the red clamp" and an LED next to the clamp or being part of the clamp may be lit red.

An interesting embodiment to ensure the correctness of the user action is to use mechanical force as the user output (claim 3). By not allowing a wrong tube connection for example, or by presenting the tube that should be connected via an actuator, erroneous user actions can be further reduced and performing the user action becomes easier and quicker for the user.

In another preferred embodiment according to claim 4, one or more user action sensors may be used to verify if the user action has been performed correctly.

Another embodiment relates to biological material sensors that allow the smart container to detect the presence of biological material (claim 5). Such a detection can be used for example in cooperation with other devices to identify which biological material is present. If a biological material is removed by a user from one container and a further container placed next to the first container receives a biological material, it can be inferred, that it may be the same material. If the user then enters an identification different from the expected identification, a warning or question can be output.

Claim 6 concerns the data security which is particularly relevant for highly regulated bioprocesses. Claim 7 further relates to documenting and preferably digitally signing the documentation of the user action by the smart cartridge creating an audit trail.

Claim 8 provides that the control unit may locally process some data thereby not depending only on a central system and possibly providing a redundancy in the overall system.

The embodiments of claim 9 relate to a central workflow management system and the coordination between the smart container and the central workflow management system. The smart consumable may be able to output the user output even if offline.

Another embodiment relates to the cooperation between two smart containers (claim 10). If a user output targets both smart containers, the smart containers may work together to provide the user guidance.

According to claim 11, the smart container may comprise a battery to ensure that it does not require a continuous supply of power from the system into which it is integrated..

The very preferred embodiment of claim 12 relates to the use of a smart container as a cartridge in an integrated bioprocessing system. Integrated bioprocessing systems are used to produce CAR T cells and the like for immunotherapy. Those systems need to perform highly specialized and individualized bioprocesses with small amounts of biological material while still being efficient. They may perform a number of unit operations, possibly on different cartridges. The history of the biological material should be documented, and errors must be avoided. Any necessary user action may be very critical if performed incorrectly while often still being necessary for tasks that are difficult to automate like clipping tubes into cartridges.

By providing user guidance via a local output arrangement of the cartridge, the overall integrated bioprocessing system may have a higher reliability.

It may further be the case that the control unit also provides at least partial information about workflow steps to be performed automatically (claim 13).

Another teaching according to claim 14, which is of equal importance, relates to an arrangement of at least two of the proposed smart containers.

Here, it is essential that the two smart containers are configured to communicate with each other.

All explanations given with regard to the proposed smart container are fully applicable.

Another teaching according to claim 15, which is of equal importance, relates to an integrated bioprocessing system for performing a bioprocess on liquid immune or naive cell cultures by performing at least one unit operation automatically, the integrated bioprocessing system utilizing at least one, in particular multiple, smart containers with physical signifiers. This second teaching concerns a use of the smart containers inside an integrated bioprocessing system and focuses on the fact that the user output, provided by the physical signifiers, can also be received by a part of the integrated bioprocessing system.

All explanations given with regard to the smart container and the proposed arrangement of at least two smart containers are fully applicable.

In the following, embodiments of the invention are explained with respect to the drawing. The drawing shows in
- Fig. 1,: two embodiments of integrated bioprocessing systems,
- Fig. 2,: smart containers for an integrated bioprocessing system,
- Fig. 3,: an empty smart container in two views,
- Fig. 4,: a smart container with a fluidic structure and a gripper gripping a tube,
- Fig. 5,: a smart container with a fluidic structure and an actuator presenting a tube and
- Fig. 6,: another smart container with a multiwell plate.

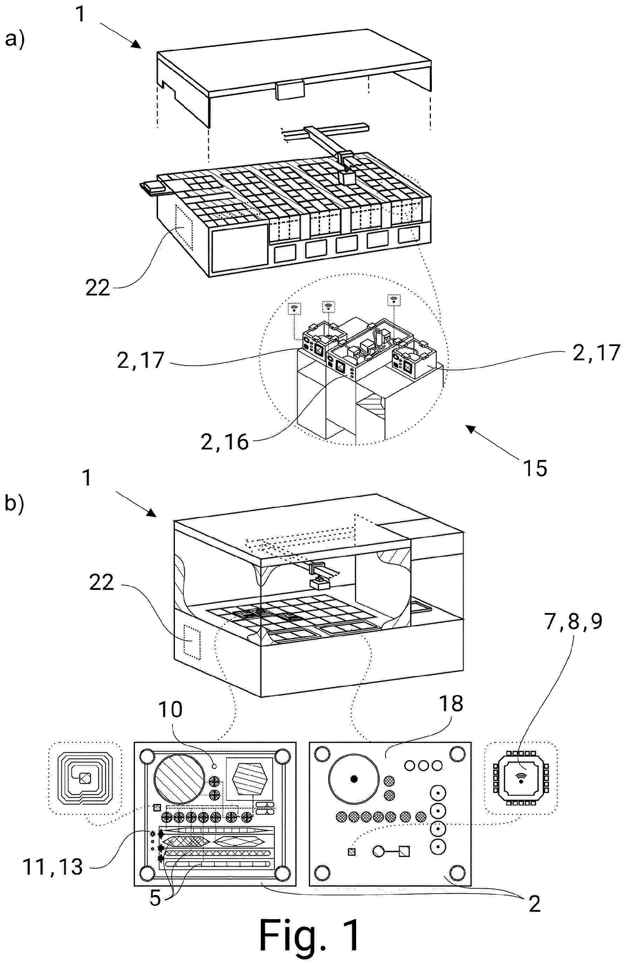

The integrated bioprocessing system 1 shown in two embodiments in Fig. 1 a) and b) is preferably adapted to perform a bioprocess for the manufacturing of genetically modified T cells. Here, T cells are genetically modified to express a chimeric antigen receptor (CAR). Consequently, the term "CAR-T cells" describes T cells that have been genetically modified to express a CAR. The genetically modified CAR-T cells, which represent the product of the bioprocess, may be administered to a patient and used to start or resume cancer treatment in the patient. As the bioprocess is performed, the initial immune cell culture is gradually processed. All explanations given are mainly directed to such a bioprocess. It may be pointed out, however, that those explanations are fully applicable to other bioprocesses as well.

The term "liquid immune cell culture" is to be understood in a broad sense and refers to an immune cell culture comprising at least one type of immune cells in any type of liquid. As will be explained below, the liquid immune cell culture may comprise other cell types that are not immune cells. Hence, the term "liquid immune cell culture" refers to a liquid immune cell culture at any stage during the bioprocess. Consequently, the type and fraction of immune cells present in the liquid immune cell culture will change during the bioprocess applied as certain immune cells are enriched or depleted from the liquid immune cell culture and/or the immune cells are genetically modified.

The term "immune cells" generally refers to different types of white blood cells. Hence, the term "immune cells" includes a variety of cells, for example, but not limited to dendritic cells, T lymphocytes, also referred to as T cells, B lymphocytes, natural killer cells, macrophages or the like. Immune cells may also include subtypes of immune cells, for example tumor-infiltrating lymphocytes or different types of T cells. Subtypes of a certain type of immune cells may be classified based on the type of antigen present at the cell surface. Hence, the term immune cells may for example refer to T cells comprising the surface antigen CD4 ("CD4+ T cells"). Typically, a certain type of immune cells, e.g., T cells, preferably a certain subtype of immune cells, e.g., CD4+ T cells, will be selectively enriched by the bioprocess, while other immune cells, e. g. macrophages, and/or other cell types that are not immune cells, e. g., erythrocytes, and/or other subtypes of immune cells, e.g., CD8+ T cells, will be depleted from the liquid immune cell culture. The immune cells to be enriched are referred to as target immune cells, all other components to be depleted from the liquid immune cell culture are referred to "impurities". Further, and as mentioned above, the target immune cells might be genetically modified.

The term "naive cells" refers to cells that can still differentiate into different target cell types. In particular, stem cells and their derivatives prior to full differentiation into a specific cell type are naive cells. The term also comprises naive immune cells.

The term "liquid" is to be understood in a broad sense as well and refers to any liquid and/or particle-containing liquid that is processed within the integrated bioprocessing system 1. Hence, the term liquid might refer to media, waste, the liquid immune cell culture, byproducts obtained during the bioprocess, samples and/or an initial immune cell culture.

Preferably, an initial immune cell culture is obtained in a process called "leukapharesis". In leukapharesis, immune cells are obtained from the patient. Additionally or alternatively, the initial immune cell culture might also be obtained from a tissue of the patient. The initial immune cell culture might also be obtained by one or more donors that are not the patient.

Depending on the source of the initial cell culture and the bioprocess to be carried out, the initial cell culture, particularly the type, amount and distribution of impurities as well as target immune or naive cells might vary.

Presently, it is preferred that any initial immune cell culture used in the bioprocess is used for only a single patient or a small number of patients, for example up to ten patients. If that is not the case then the bioprocess presently described will yield only a cell culture for a single patient or a small number of patients and cell cultures for other patients are derived from different bioprocesses. Therefore, the proposed integrated bioprocessing system 1 is used for parallelizingsmall scale bioprocesses. A bioprocess is one repetition of a certain type of bioprocess. Different bioprocesses and/or several repetitions of a bioprocess may be performed.

Proposed is a smart container 2 for performing a bioprocess and containing biological material, in particular liquid immune cell cultures. The biological material is preferably a cell culture, however, can also be any other material used in a bioprocess like a nutrient solution.

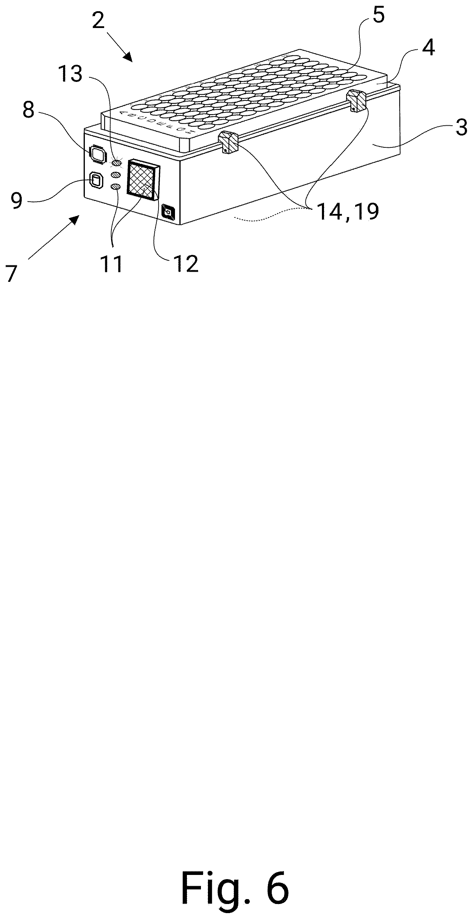

The smart container 2 comprises a base 3. Reference is made to the definition of base 3 given in the introduction. Figs. 1 to 6 show examples of smart containers 2. Figs. 1 to 5 show smart containers 2 for the integrated bioprocessing systems 1 of Fig. 1, Fig. 6 shows a smart container 2 with a multiwell plate 4.

The smart container 2 comprises on the base 3 a storage structure 5, in particular fluidic storage structure 5, for the biological material. The storage structure 5 may comprise a bag 6 or the like. In particular, the storage structure 5 may be sealed and comprise an inside that is sealed against the outside with the biological material located in the inside. Alternatively, the storage structure 5 may need to be equipped with another storage structure 5 containing the biological material. In Fig. 6, the multiwell plate 4 comprises several fluidic storage structures 5.

The smart container 2 comprises on the base 3 a control unit 7 with a processor 8 and a memory 9. The control unit 7 may be or comprise a microprocessor with or without an additional memory 9. Other processors 8, memories 9 and the like known in the art may be used. The control unit 7 or parts of it may be attached to the base 3 or embedded in the base 3. The processor 8 and/or the memory 9 and/or the control unit 7 and/or parts of the control unit 7 may be reversibly attached to the base 3. In particular, the smart container 2 may be made smart by attaching the control unit 7 or the processor 8 for example. The rest of the smart container 2 can be single-use. The rest of the container may comprise some parts of the control unit 7, in particular single-use sensors 10 and/or connections and/or a hard-coded identification or the like and may be made smart by attaching other parts of the control unit 7.

A workflow being part of the bioprocess is stored in the memory 9. The workflow may comprise one or more steps. It may comprise some instructions for the steps or a full description of the steps. Some steps may only make sense if read by other components of the integrated bioprocessing system 1. Also, an identifier of the biological material is stored in the memory 9. This may be a globally unique identifier (GUID) or a simple name or a local ID or even just the fact that the biological material is present. The workflow comprises a step with a user action performed by a user. The action to be performed by the user is therefore saved, at least temporarily, in the memory 9.

Essential is that the smart container 2 comprises a local output arrangement 11 controlled by the control unit 7, that the processor 8 of the control unit 7 generates a user output to guide the user in performing the user action and that the control unit 7 outputs the user output via the local output arrangement 11.

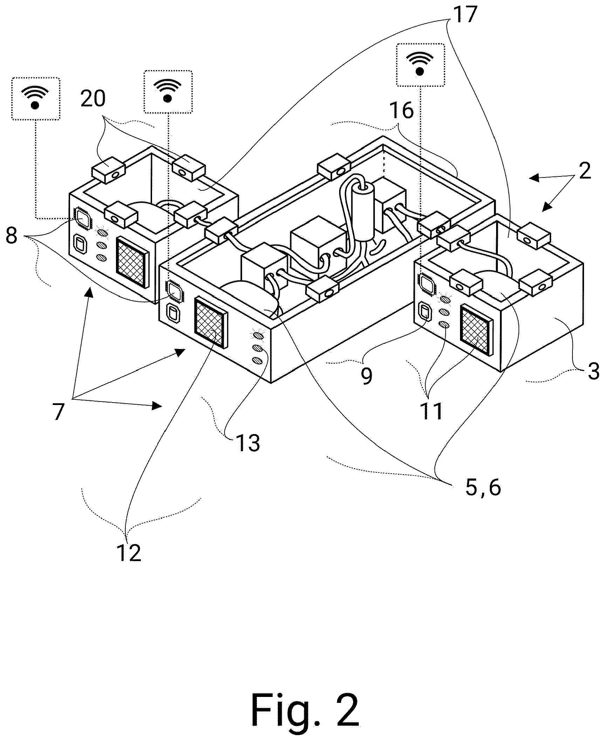

Several ways of achieving this user output will be described below. The local output arrangement 11 may include a screen 12 and/or LEDs 13 and/or a physical signifier 14 as will be explained. For example, Figs. 2 to 6 each show a display on the smart container 2. Additionally, they show different elements that may also be part of the local output arrangement 11. The local output arrangement 11 is directly controlled by the control unit 7, however, the control unit 7 may comprise multiple microprocessors for example to achieve this control.

The user performs the user action on a target, for example a tube placed inside the smart container 2. The user action may include transferring the bag 6, connecting a tube to another tube, removing or placing an element in the smart container 2, transferring the smart container 2 to a different location and the like. Here and preferably, the user action is an action on a fluidic structure inside the smart container 2, in particular an action configuring or preconfiguring the fluidic structure or connecting the fluidic structure to another fluidic structure. The user output may be an instruction to the user or an information for the user. The fluidic structure is then part of the storage structure 5.

Here and preferably, the bioprocess comprises a unit operation performed on the biological material by a unit operation device or unit operation station 15 separate from the smart container 2.

Here and preferably, the integrated bioprocessing system 1 needs the assistance of the user during the performance of a bioprocess. The integrated bioprocessing system 1 may therefore transfer the smart container 2 to a location for the user action. The smart container 2 may contain the cell culture at that point. The user may perform the user action, for example, connect the fluidic structure of the smart container 2 to the fluidic structure of another container. Anyhow, it is preferred that the smart container 2 and/or the cell culture and/or a further smart container 2 containing the cell culture returns into the integrated bioprocessing system 1 after the user action. The control unit 7 may report the completion of the user action to the integrated bioprocessing system 1.

Fig. 1 shows two embodiments of integrated bioprocessing systems 1. In Fig. 1a), cartridges 16 are stored in unit operation stations 15 and connected to receptacles 17 for the performance of unit operations. The cell cultures may be transported in receptacles 17 to other cartridges 16 for other unit operations at other unit operation stations 15. Both the receptacles 17 and the cartridges 16 may be smart containers 2.

In Fig. 1b) the integrated bioprocessing system 1 comprises standardized interfaces (not shown in detail) and individualized cartridges 16 with an adapter 18 for the standardized interfaces. The cartridge 16 itself or only in combination with the adapter 18 may be a smart container 2. This is particularly interesting as the cartridge 16 without the adapter 18 may be single-use and the adapter 18 multi-use. The control unit 7 may be arranged partly or completely on the adapter 18.

In a preferred embodiment the local output arrangement 11 is arranged on the base 3 as shown in all figures. Nevertheless and equally preferred, the local output arrangement 11 may be temporarily connected to the base 3. For example, the smart container 2 may be connected to a docking station comprising the local output arrangement 11. The docking station may then be controlled by the control unit 7.

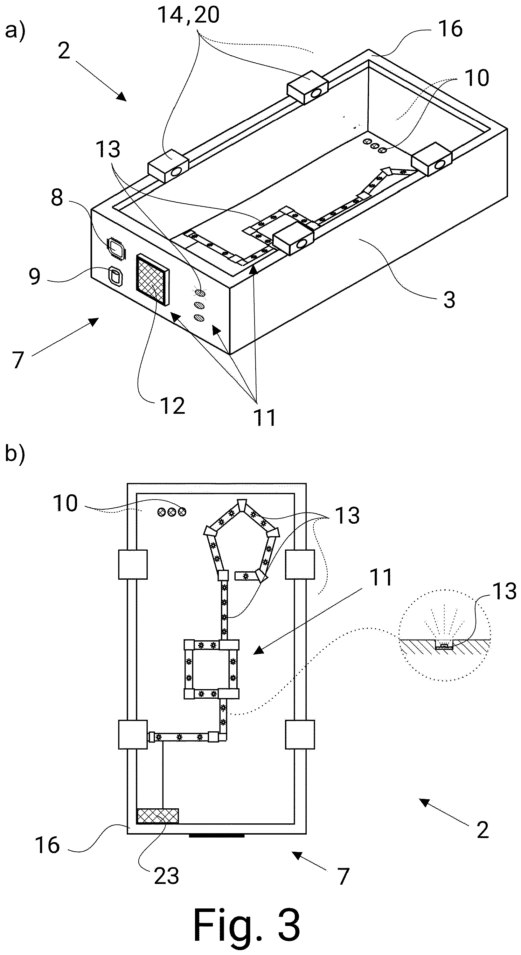

Here and preferably, the local output arrangement 11 comprises a screen 12 and/or one or more LEDs 13. As can for example be seen in the empty smart container 2 in Fig. 3, the control unit 7 may output the user output via one or more LEDs 13 placed near a target of the user action. In Fig. 3, the LEDs 13 are placed below locations where the fluidic structure for the cartridge 16 should be placed. In particular, the integrated bioprocessing system 1 may use preconfigured cartridges 16 to perform the unit operations. The preconfiguration may be a manual task. By instructing the user via LEDs 13, the preconfiguration can be completed quicker and without errors. Other light emitting elements may be used instead of LEDs 13.

Multiple LEDs 13 may be present on the base 3, preferably only a subset is activated for one user action. The LEDs 13 may for example start red at a location of a sensor 10 and turn green when the sensor 10 is detected.

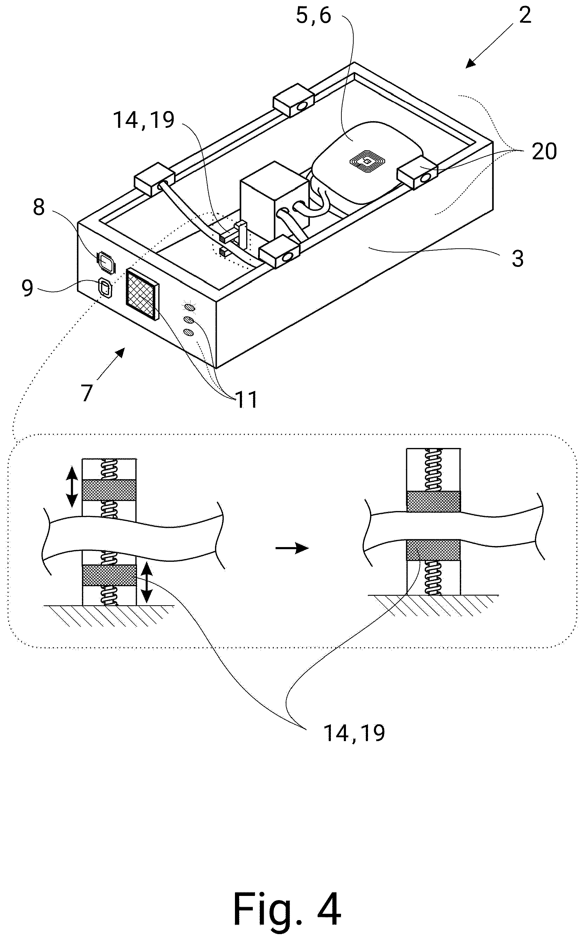

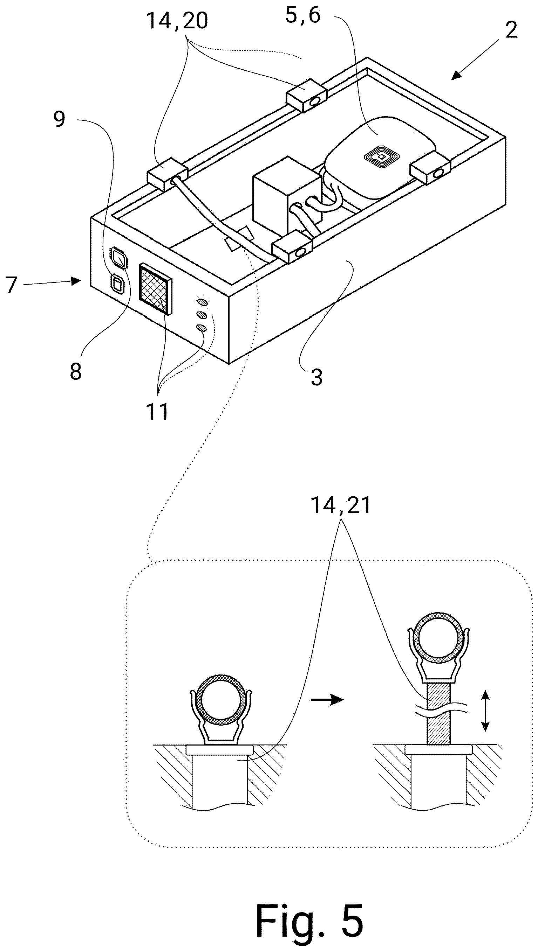

According to one embodiment it is proposed, that the smart container 2 comprises a physical signifier 14 and that the control unit 7 controls the physical signifier 14 to use mechanical force to output the user output. It may be the case that the physical signifier 14 releases a target of the user action or holds an element that is not target of the user action. Fig. 4 shows a gripper 19 gripping a tube. The gripper 19 may instead release the tube or hold a tube that should not be acted on by the user. Here and preferably, the physical signifier 14 is a gripper 19 (Fig. 4) or a clamp 20 (integrated into the holders of the cartridges 16 in the figures) or an actuator for presentation 21 of the target of the user action (Fig. 5). As can be seen, combinations of the physical signifiers 14 may be present. It may also be the case that an actuator necessary for performing the bioprocess via the integrated bioprocessing system 1 is used as a physical signifier 14. For example, the integrated bioprocessing system 1 may include an automatic way of connecting cartridges 16 and receptacles 17, for example by tube welding. The automatic connection may necessitate the clamping of the tubes and the same clamps 20 may be used as physical signifiers 14 in a different situation.

The physical signifier 14 may release a tube or open a space for a tube or the like. The target can be part of the physical signifier 14 or part of the storage structure 5.

It may also be the case that a physical signifier 14 not part of the local output arrangement 11 is connected to the smart container 2 and indirectly controlled by the control unit 7, for example by sending a request to another control unit 7.

It may be the case that the smart container 2 comprises one or more user action sensors 10 and that the control unit 7 verifies, based on reading of the one or more user action sensors 10, if the user action has been performed correctly. A user action sensor 10 may be integrated into the physical signifier 14 and detect the presence of the tube. Figs. 4 and 5 show RFID circuits on the storage structure 5, here the bags 6. The control unit 7 may automatically read the RFID circuits or other identifiers of an element added by a user.

It is preferred, that the control unit 7 initiates a deviation routine if the action has not been performed correctly and/or initiates the next step of the workflow if the action has been performed correctly. The deviation routine may include a notification to a user via the local output arrangement 11, like flashing an LED 13 in red, and/or stopping or inhibiting a next workflow step, for example by not releasing a gripper 19 or clamp 20 or by communicating with the integrated bioprocessing system 1. Initiating the next step may include displaying a next step by the local output arrangement 11 or communicating with the integrated bioprocessing system 1 such that the integrated bioprocessing system 1 initiates an automatic next step.

It may also be the case, that the control unit 7 outputs an alerting user output in the case of an alarm state, for example by flashing a light. Usually, if a machine has an issue, a central alarm is raised, which potentially then directs the user to a point of intervention via a central user interface or the like. This means that the user needs to go through several steps to get to the problem component.

In the case of the smart container 2, which has the local output arrangement 11 capable of drawing attention to itself, attention can be drawn to the point of action and the issue attended to more rapidly. This is especially advantageous in time-critical bioprocesses.

Further, for example in the case where a smart container 2 detects a leak in itself, the alert may be time-critical. The slower the intervention is, the more material, for example the liquid immune cell culture, is lost and the greater the risk of a contamination event. The smart container 2 capable of drawing attention to itself decreases the risk of contaminating the material and thereby reduces the risk of the bioprocess failing and/or additional patient material being needed.

Further, in some cases a fault, exemplarily a loss of power, may affect the integrated bioprocessing system 1 as such and therefore the integrated bioprocessing system 1 may be unable to tell the user where they should attend. Many of the liquid immune cell cultures within the system will be in a good state until the error is fixed (for example, because they are in a relatively stable temperature and do not require intervention). But some of the smart containers 2 may be in the middle of time critical operations, for example, because the liquid immune cell culture contained in a smart container 2 needs to be transported between two unit operations. These smart containers 2 can draw attention to themselves for immediate user intervention, thereby keeping all the smart containers 2 "safe" until the main fault is resolved.

In general, the control unit 7 may also detect a wrong workflow step performed by the user or automatically for example by communicating with the integrated bioprocessing system 1 or by detecting devices, elements or the like in its environment. The control unit 7 may react with the deviation routine to a wrong workflow.

According to one embodiment it is proposed, that the smart container 2 comprises one or more biological material sensors 10 and that the control unit 7 detects, based on the one or more biological material sensors 10, if one or more biological materials are present. The identifier described may be used additionally or alternatively to identify a biological material. By knowing for example which liquid immune cell culture is placed into the cartridge 16, a compliant bioprocess can be, in particularly redundantly, ensured and/or documented by the smart container 2.

The one or more biological material sensors 10 comprise an optical sensor 10 and/or a capacitance sensor 10. Those may be used to detect only the presence of any biological material, too.

The control unit 7 may comprise a tamperproof element, in particular a trusted platform module. The memory 9 may be encrypted. The control unit 7 may comprise a private key for digitally signing data. Naturally, tamperproofness will never be absolute. However, for example, the tamperproof element may be protected or even placed such that tampering with it would include destroying the smart cartridge 16 and/or the storage structure 5.

For documentation purposes, the control unit 7 may document the user action and store the documentation or send the documentation for storage to another system. Generally, the control unit 7 may be used to generate a primary or redundant documentation of the bioprocess, steps of the bioprocess or the workflow. It may be the case that the control unit 7 digitally signs the documentation. This ensures a digital trail for the physical biological material.

It will be described below how different smart containers 2 may work together. For that case and in general it is preferred, that the control unit 7 signs data it has generated itself together with data it received from another device, in particular from another smart container 2. Two smart containers 2 may also sign the other smart container's 2 data after an interaction. In this way, the way of a sample even through different smart containers 2 may be trailed digitally.

According to one embodiment it is proposed, that the control unit 7 locally processes readings of one or more sensors 10 and/or data communicated from a unit operation device and the workflow to derive the user output. By making the smart container 2 participate in the generation of the user output, different knowledge levels may be implemented in the integrated bioprocessing system 1. In particular, for example with the embodiment of Fig. 1b), the integrated bioprocessing system 1 may not know everything about the respective unit operations. It may even be the case that the workflow stored in the memory 9 includes substantial parts of the bioprocess to be performed by the integrated bioprocessing system 1. Therefore, the control unit 7 may control part of the integrated bioprocessing system 1 indirectly by providing workflow steps to be performed or even directly.

As an alternative, the control unit 7 communicates with a central workflow management system 22 and the control unit 7 receives one or more next steps from the central workflow management system 22 and updates the workflow accordingly. Preferably, the control unit 7 is then adapted to output the user output while not having a connection to the central workflow management system 22. Such offline capability may be particularly relevant for time-critical bioprocesses.

According to one embodiment it is proposed, that the control unit 7 is adapted to communicate with a control unit 7 of another smart container 2. This embodiment is particularly interesting if the cell culture or another biological material is transferred between the two smart containers 2.

Preferably, the control unit 7 is adapted to work with the control unit 7 of the other smart container 2 to output a combined user output, in particular for a user action targeting both smart containers 2. As such, the smart containers 2 may both light an LED 13 on a tube connection point each and the user may connect the tubes assigned to the tube connection points. Fig. 2 shows such a tube connection in a connected state between a cartridge 16 and two receptacles 17.

It may also be the case that the control unit 7 comprises a battery 23. In particular, the control unit 7 may be completely powered by the battery 23 at times. For example, the battery 23 may be charged only during a storage of the smart container 2 or not at all. Preferably, the smart container 2 comprises on the base 3 a charging connector, in particular socket or plug, for charging the battery 23, preferably while a unit operation is performed on the biological material, in particular performed inside the smart container 2. Charging the smart container 2 during use enables an easy reuse of the smart container 2. As, preferably, the smart container 2 is driven by the integrated bioprocessing system 1 to perform a unit operation, for example if the smart container 2 contains a centrifuge head or pump head, providing for a charging connector, too, is not problematic. A further preferred embodiment for charging is wireless charging. Preferably, the smart container 2 comprises on the base 3 a charging interface for wireless charging.

The charging interface may comprise an inductor for receiving electrical energy and charging the battery 23.

As has been extensively explained, in one embodiment, the smart container 2 is a cartridge 16 for an integrated bioprocessing system 1 for performing a bioprocess in liquid immune or naive cell cultures.

Preferably, the cartridge 16 is preconfigured with a fluidic structure. The fluidic structure may contain an active element for performing a unit operation, like a centrifuge head. The fluidic structure may be single-use.

The integrated bioprocessing system 1 may perform at least part of a unit operation in the cartridge 16, in particular in the fluidic structure of the cartridge 16.

The liquid immune or naive cell cultures may be transferred from one smart container 2 to another smart container 2, in particular automatically by the integrated bioprocessing system 1.

Preferably, the user action is part of the preconfiguration and/or the transfer.

It may be the case that the smart container 2 is used to transport the cell culture out of the integrated bioprocessing system 1 and possibly to a final destination. It may then be the case that the control unit 7 stores a documentation of a part or of the full bioprocess performed by the integrated bioprocessing system 1. The documentation may be in part generated by the integrated bioprocessing system 1 and/or by the control unit 7. The documentation or parts of it may be shown on the screen 12 after the smart container 2 left the integrated bioprocessing system 1. The smart container 2 may further measure and document aspects of transport of the smart container 2, for example a temperature and/or an acceleration.

According to one embodiment it is proposed, that the control unit 7 communicates a workflow step to a unit operation device for performing the unit operation with the biological material. As described above, the control unit 7 may therefore indirectly control part of the bioprocess.

Another teaching which is of equal importance relates to an arrangement of at least two smart containers 2 each configured according to the first teaching.

According to that teaching it is proposed, that the two smart containers 2 are configured to communicate with each other.

Another teaching which is of equal importance relates to an integrated bioprocessing system 1 for performing a bioprocess on liquid immune or naive cell cultures by performing at least one unit operation automatically, the integrated bioprocessing system 1 utilizing at least one, in particular multiple, smart containers 2, wherein the smart container 2 is adapted for performing a bioprocess and containing biological material, in particular liquid immune cell cultures, wherein the smart container 2 comprises a base 3, wherein the smart container 2 comprises on the base 3 a storage structure 5, in particular fluidic storage structure 5, for the biological material, wherein the smart container 2 comprises on the base 3 a control unit 7 with a processor 8 and a memory 9, wherein a workflow being part of the bioprocess is stored in the memory 9, wherein an identifier of the biological material is stored in the memory 9, wherein the workflow comprises a step with a user action performed by a user. It is essential for this teaching, that the smart container 2 comprises a local output arrangement 11 controlled by the control unit 7, that the smart container 2 comprises a physical signifier 14, that the control unit 7 controls the physical signifier 14 to use mechanical force to output a guiding output, in particular a user output. All explanations given with regard to the smart container may apply. In particular, all explanations given with regard to the physical signifier may apply and the user output may be the guiding output instead.

A key aspect of integrated bioprocessing systems 1 for performing a bioprocess on liquid immune or naive cell cultures is that they should protect the patient material even in the event of a system failure. The smart container 2 may not only process data locally, but may also perform some operations on the material it contains and/or relative to the integrated bioprocessing system 1, in particular in the event of a failure at the level of the whole system. Preferably, the container performs at least one operation to protect the biological material it contains, in particular in case of a failure of the integrated bioprocessing system 1. For example, the smart container 2 may control temperature and/or agitate and/or add a liquid to the biological material autonomously, without requiring step by step instructions from the integrated bioprocessing system 1.

## Claims

1. Smart container for performing a bioprocess and containing biological material, in particular liquid immune cell cultures, wherein the smart container (2) comprises a base (3), wherein the smart container (2) comprises on the base (3) a storage structure (5), in particular fluidic storage structure (5), for the biological material,
wherein the smart container (2) comprises on the base (3) a control unit (7) with a processor (8) and a memory (9), wherein a workflow being part of the bioprocess is stored in the memory (9), wherein an identifier of the biological material is stored in the memory (9), wherein the workflow comprises a step with a user action performed by a user,
**characterized in**
**that** the smart container (2) comprises a local output arrangement (11) controlled by the control unit (7), that the processor (8) of the control unit (7) generates a user output to guide the user in performing the user action and that the control unit (7) outputs the user output via the local output arrangement (11).

2. Smart container according to claim 1, **characterized in that** the local output arrangement (11) is arranged on the base (3), or, that the local output arrangement (11) is temporarily connected to the base (3), preferably, that the local output arrangement (11) comprises a screen (12) and/or one or more LEDs (13), more preferably, that the control unit (7) outputs the user output via one or more LEDs (13) placed near a target of the user action.

3. Smart container according to claim 1 or 2, **characterized in that** the smart container (2) comprises a physical signifier (14), that the control unit (7) controls the physical signifier (14) to use mechanical force to output the user output, preferably, that the physical signifier (14) releases a target of the user action or holds an element that is not target of the user action, more preferably, that the physical signifier (14) is a gripper (19) or a clamp (20) or an actuator for presentation (21) of the target of the user action.

4. Smart container according to one of the preceding claims, **characterized in that** the smart container (2) comprises one or more user action sensors (10), that the control unit (7) verifies, based on readings of the one or more user action sensors (10), if the user action has been performed correctly, preferably, that the control unit (7) initiates a deviation routine if the action has not been performed correctly and/or initiates the next step of the workflow if the action has been performed correctly.

5. Smart container according to one of the preceding claims, **characterized in that** the smart container (2) comprises one or more biological material sensors (10), that the control unit (7) detects, based on the one or more biological material sensors (10), if one or more biological materials are present, preferably, that the one or more biological material sensors (10) comprise an optical sensor (10) and/or a capacitance sensor (10).

6. Smart container according to one of the preceding claims, **characterized in that** the control unit (7) comprises a tamperproof element, in particular a trusted platform module, and/or, that the memory (9) is encrypted, and/or, that the control unit (7) comprises a private key for digitally signing data.

7. Smart container according to one of the preceding claims, **characterized in that** the control unit (7) documents the user action and stores the documentation or sends the documentation for storage to another system, preferably, that the control unit (7) digitally signs the documentation.

8. Smart container according to one of the preceding claims, **characterized in that** the control unit (7) locally processes readings of one or more sensors (10) and/or data communicated from a unit operation device and the workflow to derive the user output.

9. Smart container according to one of the preceding claims, **characterized in that** the control unit (7) communicates with a central workflow management system (22), that the control unit (7) receives one or more next steps from the central workflow management system (22) and updates the workflow accordingly, preferably, that the control unit (7) is adapted to output the user output while not having a connection to the central workflow management system (22).

10. Smart container according to one of the preceding claims, **characterized in that** the control unit (7) is adapted to communicate with a control unit (7) of another smart container (2), preferably, that the control unit (7) is adapted to work with the control unit (7) of the other smart container (2) to output a combined user output, in particular for a user action targeting both smart containers (2).

11. Smart container according to one of the preceding claims, **characterized in that** the control unit (7) comprises a battery (23), in particular is completely powered by the battery (23) at times, preferably, that the smart container (2) comprises on the base (3) a charging connector, in particular socket or plug, for charging the battery (23), preferably while a unit operation is performed on the biological material, in particular inside the smart container (2).

12. Smart container according to one of the preceding claims, **characterized in that** the smart container (2) is a cartridge (16) for an integrated bioprocessing system (1) for performing a bioprocess in liquid immune or naive cell cultures, preferably, that the cartridge (16) is preconfigured with a fluidic structure, and/or, that the integrated bioprocessing system (1) performs at least part of a unit operation in the cartridge (16), in particular in the fluidic structure of the cartridge (16), and/or, that the liquid immune or naive cell cultures are transferred from one smart container (2) to another smart container (2), in particular automatically by the integrated bioprocessing system (1), preferably, that the user action is part of the preconfiguration and/or the transfer.

13. Smart container according to one of the preceding claims, **characterized in that** the control unit (7) communicates a workflow step to a unit operation device for performing the unit operation with the biological material.

14. Arrangement of at least two smart containers (2) each configured according to one of the preceding claims, **characterized in that** the two smart containers (2) are configured to communicate with each other.

15. Integrated bioprocessing system for performing a bioprocess on liquid immune or naive cell cultures by performing at least one unit operation automatically, the integrated bioprocessing system (1) utilizing at least one, in particular multiple, smart containers (2),
wherein the smart container (2) is adapted for performing a bioprocess and containing biological material, in particular liquid immune cell cultures, wherein the smart container (2) comprises a base (3), wherein the smart container (2) comprises on the base (3) a storage structure (5), in particular fluidic storage structure (5), for the biological material,
wherein the smart container (2) comprises on the base (3) a control unit (7) with a processor (8) and a memory (9), wherein a workflow being part of the bioprocess is stored in the memory (9), wherein an identifier of the biological material is stored in the memory (9), wherein the workflow comprises a step with a user action performed by a user,
**characterized in**
**that** the smart container (2) comprises a local output arrangement (11) controlled by the control unit (7),
**that** the smart container (2) comprises a physical signifier (14), that the control unit (7) controls the physical signifier (14) to use mechanical force to output a guiding output, in particular a user output.
